# EUROPEAN PATENT SPECIFICATION

(11) **EP 3 986 300 B1**
(45) Date of publication and mention of the grant of the patent: **27.11.2024**
(21) Application number: 20731568.0
(22) Date of filing: 02.06.2020
(51) Int. Cl.: A61B 17/34, A61B 17/16, A61B 17/17, A61B 90/00, A61M 5/158

(54) **BONE-PENETRATING MANUAL DRIVER AND STABILIZER ASSEMBLY FOR INTRAOSSEOUS ACCESS**
KNOCHENDURCHDRINGENDE MANUELLE FAHRER- UND STABILISATORANORDNUNG FÜR DEN INTRAOSSÄREN ZUGANG
ENSEMBLE DISPOSITIF D'ENFONCEMENT MANUEL DE PÉNÉTRATION OSSEUSE ET STABILISATEUR POUR ACCÈS INTRA-OSSEUX

(30) Priority: 22.06.2019 US 201962865175 P
(43) Date of publication of application: 27.04.2022
(73) Proprietor: Teleflex Life Sciences II LLC, Wilmington, Delaware 19808 (US)
(72) Inventor: TIERNEY, Morgan, Ferbane Co Offaly (IE); FOMINAS, Aleksejus, Athlone Co Roscommon (IE); TREXLER, Wade Kevin, Coopersburg, Pennsylvania 18036 (US); HEINLY, Kurt Donald, Wernersville, Pennsylvania 19565 (US); ROWE, David Troy, Fleetwood, Pennsylvania 19522 (US); HUHN, Stephan M., Manheim, Pennsylvania 17545 (US)
(74) Representative: Jacob, Reuben Ellis
(86) International application number: PCT/IB2020/055198
(87) International publication number: WO 2020/260993

(56) References cited:
- US-A1- 2010 312 246
- US-A1- 2010 312 246
- US-B2- 10 136 878
- US-B2- 10 136 878

## Description

### CROSS-REFERENCE TO RELATED APPLICATIONS

The present application claims the benefit of priority to U.S. Provisional Patent Application No. 62/865,175, filed June 22, 2019.

### TECHNICAL FIELD

The present disclosure generally relates to a medical apparatus for locating and accessing an intraosseous space of a patient. More specifically, the present disclosure relates to a bone-penetrating manual driver and stabilizer assembly for placement of a conduit into the intraosseous space within a bone of a patient.

### BACKGROUND

Many life-threatening emergencies, including shock, trauma, cardiac arrest, drug overdoses, diabetic ketoacidosis, arrhythmias, burns, and status epilepticus, just to name a few, often unnecessarily result in death because intravenous (IV) access cannot be achieved in a timely manner. An essential element for treating many life threatening emergencies is the rapid establishment of an IV line in order to administer drugs and fluids directly into a patient's vascular system. Whether in an ambulance by paramedics, in an emergency room by emergency specialists or on a battlefield by an Army medic, the goal is the same--quickly start an IV in order to administer lifesaving drugs and fluids. To a large degree, ability to successfully treat most critical emergencies is dependent on the skill and luck of an operator in accomplishing vascular access. While relatively easy to start an IV on some patients, doctors, nurses and paramedics may nevertheless experience difficulty establishing IV access in some patients. The success rate on the battlefield may be much lower, in which wounded soldiers are often probed repeatedly with sharp needles in an attempt to quickly establish IV access.

In the case of patients with chronic disease or the elderly, availability of easily accessible veins may be depleted. Other patients may have no available IV sites due to anatomical scarcity of peripheral veins, obesity, extreme dehydration or previous IV drug use. For such patients, finding a suitable site for administering lifesaving therapy often becomes a monumental and frustrating task. As a result, patients with life threatening emergencies may die when access to the vascular system with lifesaving IV therapy is delayed or simply not possible.

There are various circumstances under which it is desirable to introduce drugs or other liquids into the marrow of a subject's bone. For example, in cases where a subject has suffered from serious trauma or cardiac arrest it may not be practical to deliver liquids by way of intravenous (IV) infusions. Intraosseous infusion may also be useful for delivering fluids to newborns and small children in which suitable blood vessels are difficult to access. Intraosseous infusion may be used to deliver fluids into a subject's sternum, humerus, femur, tibia, or other bone. Intraosseous infusion has the advantage that, with appropriate technology, a pathway for intraosseous infusion can be established very rapidly. This can save lives in critical situations. Portals in bone may also be applied to withdraw or aspirate fluid from within the bone.

The intraosseous (IO) space provides a direct conduit to a patient's vascular system and provides an attractive alternate route to administer IV drugs and fluids. Drugs administered intraosseously enter a patient's blood circulation system rapidly, thus bone marrow may function as a large non-collapsible vein.

Proper placement of an intraosseous needle in the bone is critical. If a user attempts to insert the needle in the wrong place, the bone might be too thick and therefore difficult for the needle to penetrate. Alternatively, the bone might be too thin, in which case the needle could completely penetrate the anterior and posterior sides of the bone, thus missing the intraosseous region entirely. Also, placing the needle at an angle that is not substantially perpendicular to the surface of the bone may lead to the needle breaking, or other complications. Furthermore, certain powered drivers are unable to successfully penetrate bone when their respective power source is depleted. Additionally, the sharp penetrator tips of conventional driver assemblies can be dangerous if they are accidentally mishandled by a user prior to a planned insertion procedure. For instance, without adequate sharps protection, the user is susceptible to accidentally poking himself or another individual with the penetrator.

Documents US 2010/312246 A1 and US 10136878 B2 disclose examples of intraosseus access devices in the prior art.

Therefore, a need exists for a bone-penetrating manual driver and stabilizer assembly operable to locate a suitable insertion site and provide a quick and easy conduit to an intraosseous space within a bone of a patient. There is a further need for a bone-penetrating manual driver and stabilizer assembly having a first mode of operation for sternal insertion, and a second mode of operation for peripheral insertion.

### SUMMARY

Claim 1 defines the invention and dependent claims disclose embodiments. No surgical methods are claimed. The foregoing needs are met by implementations of an apparatus for accessing an intraosseous space within a bone of a patient according to the present disclosure. According to one aspect of the disclosure, the apparatus comprises a manual intraosseous driver including a handle and a penetrator assembly, the penetrator assembly having a sharp penetrating end configured to penetrate a bone and associated bone marrow; and a stabilizer including a retainer and a stabilizer housing; the retainer having a first retainer end, a second retainer end, and an internal passageway extending from the first retainer end to the second retainer end, the internal passageway configured to removably receive a portion of the manual intraosseous driver; and the stabilizer housing having a first housing end, a second housing end, and an internal housing section configured to receive a portion of the retainer; where the penetrator assembly is operable to provide access to a sternal intraosseous space when the manual driver is coupled to the stabilizer in a first mode of operation; and where the penetrator assembly is operable to provide access to a peripheral intraosseous space when the manual driver is decoupled from the stabilizer in a second mode of operation.

According to another aspect of the disclosure, the stabilizer further comprises a protective shield slidably disposed in the internal housing section of the stabilizer housing, the protective shield configured to move between an extended position and a retracted position during the first mode of operation.

According to another aspect of the disclosure, the extended position of the protective shield is operable to provide sharps protection from the sharp penetrating end of the penetrating assembly, and the retracted position of the protective shield is operable to expose the sharp penetrating end of the penetrating assembly to permit insertion of the penetrating assembly into the bone and associated bone marrow.

According to another aspect of the disclosure, the protective shield includes a first shield end slidably coupled to the second retainer end of the retainer.

According to another aspect of the disclosure, the stabilizer further comprises a bone probe ring having a first ring end, a second ring end coupled to the first housing end of the stabilizer housing, and a bone probe extending from the second ring end, the bone probe including a bone probe tip operable to penetrate skin and subcutaneous tissue.

According to another aspect of the disclosure, the protective shield includes a longitudinal channel configured to slidably receive the bone probe.

According to another aspect of the disclosure, the bone probe tip of the bone probe is disposed within the longitudinal channel to provide sharps protection when the protective shield is in the extended position, and wherein the bone probe tip of the bone probe extends from the longitudinal channel when the protective shield is in the retracted position.

According to another aspect of the disclosure, the stabilizer further comprises an outer sleeve slidably coupled to the bone probe ring, the outer sleeve operable to move from an undeployed position to a deployed position in the first mode of operation.

According to another aspect of the disclosure, the undeployed position of the outer sleeve permits the penetrator assembly to penetrate the skin and subcutaneous tissue, and where the deployed position of the outer sleeve permits the penetrator assembly to penetrate the bone and associated bone marrow.

According to another aspect of the disclosure, the outer sleeve includes a first detent and a second detent, the first detent spaced apart from the second detent.

According to another aspect of the disclosure, the bone probe ring is configured to releasably engage the first detent when the outer sleeve is in the undeployed position, and wherein the bone probe ring is configured to releasably engage the second detent when the outer sleeve is in the deployed position.

According to another aspect of the disclosure, the first ring end of the bone probe ring comprises a resilient arm including and outwardly protruding catch operable to engage the first detent when the outer sleeve is in the undeployed position and operable to engage the second detent when the outer sleeve is in the deployed position.

According to another aspect of the disclosure, the stabilizer further comprises a stabilizing base connected to the protective shield.

According to another aspect of the disclosure, the stabilizing base comprises a guide hole configured to guide the penetrator assembly during the first mode of operation.

According to another aspect of the disclosure, the stabilizing base comprises a through-hole aligned with the longitudinal channel of the protective shield, the through-hole configured to permit passage of the bone probe through the base during the first mode of operation.

According to another aspect of the disclosure, the stabilizing base comprises an alignment cutout operable to indicate placement of the stabilizing base against the patient's sternum during the first mode of operation.

According to another aspect of the disclosure, the intraosseous access devices comprises a safety latch operable to prevent the outer sleeve from moving from the undeployed position to the deployed position during the first mode of operation, and operable to prevent the protective shield from moving from the extended position to the retracted position during the first mode of operation.

According to another aspect of the disclosure, the first retainer end of the retainer is configured to releasably secure the manual intraosseous driver to the stabilizer.

According to another aspect of the disclosure, the manual intraosseous driver further comprises an activator, where the activator is operable to move to a locked position to lock the manual intraosseous driver to the stabilizer for intraosseous insertion of the penetrator assembly in the first mode of operation, and where the activator is operable to move to an unlocked position to unlock the manual intraosseous driver from the stabilizer for intraosseous insertion of the penetrator assembly in the second mode of operation.

According to another aspect of the disclosure, the manual intraosseous driver further comprises a collar including a collar aperture configured to partially receive a retention member when the activator is moved to the locked position.

According to another aspect of the disclosure, the manual intraosseous driver further comprises a biasing member configured to bias the activator toward the locked position.

According to another aspect of the disclosure, the penetrator assembly comprises an inner penetrator hub having a first end and a second end, the first end of the inner penetrator hub coupled to the handle, and the second end of the inner penetrator hub coupled to an inner penetrator; an outer penetrator hub having a first end and a second end, the first end of the outer penetrator hub releasably engaged to the second end of the inner penetrator hub, and the second end of the outer penetrator hub coupled to an outer penetrator defining a longitudinal hollow bore configured to slidably receive the inner penetrator.

According to another aspect of the disclosure, the inner penetrator comprises a rigid stylet.

According to another aspect of the disclosure, the outer penetrator comprises a flexible cannula.

According to another aspect of the disclosure, the handle includes an ergonomic grip suitable for grasping during the first and second modes of operation.

According to another aspect of the disclosure, the handle is configured to allow manual force to be applied and at the same time permit rotation of the handle during intraosseous insertion of the penetrator assembly.

According to another aspect of the disclosure, a method for accessing an intraosseous space of a patient comprises providing an intraosseous access device comprising a manual intraosseous driver coupled to a stabilizer, the manual intraosseous driver including a handle and a penetrator assembly, the stabilizer including a retainer having an internal passageway, and the penetrator assembly slidably received within the internal passageway and having a sharp penetrating end configured to penetrate a bone and associated bone marrow; determining a mode of operation of the intraosseous access device based on a location of a target site for intraosseous access; positioning the stabilizer over a patient's sternum and manually inserting the penetrator assembly into a sternal intraosseous space when the mode of operation is a first mode of operation; and decoupling the manual intraosseous driver from the stabilizer and manually inserting the penetrator assembly into a peripheral intraosseous space when the mode of operation is a second mode of operation.

According to another aspect of the disclosure, the stabilizer further comprises a protective shield movable between an extended position and a retracted position during the first mode of operation, where the extended position of the protective shield provides sharps protection from the sharp penetrating end of the penetrating assembly, and where the retracted position of the protective shield exposes the sharp penetrating end of the penetrating assembly to permit insertion of the penetrating assembly into the bone and associated bone marrow.

According to another aspect of the disclosure, the stabilizer further comprises a bone probe ring including a bone probe having a bone probe tip operable to penetrate skin and subcutaneous tissue, wherein the protective shield provides sharps protection from the bone probe tip when the protective shield is in the extended position, and wherein the protective shield exposes the bone probe tip of the bone probe when the protective shield is in the retracted position.

According to another aspect of the disclosure, the stabilizer further comprises an outer sleeve slidably coupled to the bone probe ring and movable from an undeployed position to a deployed position in the first mode of operation, where the penetrator assembly penetrates the skin and subcutaneous tissue when the outer sleeve is in the undeployed position, and where the penetrator assembly penetrates the bone and associated bone marrow when the outer sleeve is in the deployed position.

According to another aspect of the disclosure, the stabilizer further comprises a stabilizing base including a guide hole for guiding the penetrator assembly during the first mode of operation.

According to another aspect of the disclosure, the manual intraosseous driver further comprises an activator movable between a locked position that locks the manual intraosseous driver to the stabilizer for intraosseous insertion of the penetrator assembly in the first mode of operation, and an unlocked position that unlocks the manual intraosseous driver from the stabilizer for intraosseous insertion of the penetrator assembly in the second mode of operation.

According to another aspect of the disclosure, the penetrator assembly comprises an inner penetrator hub having a first end and a second end, the first end of the inner penetrator hub coupled to the handle, and the second end of the inner penetrator hub coupled to an inner penetrator; an outer penetrator hub having a first end and a second end, the first end of the outer penetrator hub releasably engaged to the second end of the inner penetrator hub, and the second end of the outer penetrator hub coupled to an outer penetrator defining a longitudinal hollow bore configured to slidably receive the inner penetrator.

According to another aspect of the disclosure, the inner penetrator comprises a rigid stylet.

According to another aspect of the disclosure, the outer penetrator comprises a flexible cannula.

There has thus been outlined certain aspects of the disclosure in order that the detailed description thereof may be better understood, and in order that the present contribution to the art may be better appreciated. There are additional implementations of the disclosure that will be described below and which form the subject matter of the claims appended hereto.

In this respect, before explaining at least one aspect of the intraosseous access device in detail, it is to be understood that the apparatus is not limited in its application to the details of construction and to the arrangements of the components set forth in the following description or illustrated in the drawings. The intraosseous access device is capable of aspects in addition to those described, and of being practiced and carried out in various ways. Also, it is to be understood that the phraseology and terminology employed herein, as well as the abstract, are for the purpose of description and should not be regarded as limiting.

As such, those skilled in the art will appreciate that the conception upon which this disclosure is based may readily be utilized as a basis for the designing of other structures, methods, and systems for carrying out the several purposes of the intraosseous access device. The scope of the invention is solely defined by the claims.

### BRIEF DESCRIPTION OF THE DRAWINGS

In order that the disclosure may be readily understood, aspects of the intraosseous (IO) access device are illustrated by way of examples in the accompanying drawings, in which like parts are referred to with like reference numerals throughout.
FIG. 1 illustrates a schematic view of a ribcage of a human.
FIG. 2 illustrates a cross-section view of a region of the sternum of a human.
FIG. 3 illustrates a perspective view of an intraosseous access device according to the present disclosure.
FIG. 4 illustrates an exploded perspective view of the intraosseous device according to the present disclosure.
FIG. 5 illustrates a side elevation view of the intraosseous device according to the present disclosure.
FIG. 6 illustrates a cross-sectional side elevation view of the intraosseous device according to the present disclosure prior to use.
FIG. 7A illustrates a perspective view of an implementation of a bone probe in accordance with the present disclosure.
FIG. 7B illustrates a side view of the bone probe of FIG. 7A.
FIG. 7C illustrates a perspective view of another implementation of a bone probe in accordance with the present disclosure.
FIG. 7D illustrates a side view of the bone probe of FIG. 7C.
FIG. 8 illustrates a cross-sectional side elevation view of the intraosseous device according to the present disclosure during use in a first mode of operation.
FIG. 9 illustrates a cross-sectional side elevation view of the intraosseous device according to the present disclosure during use in a first mode of operation.
FIG. 10 illustrates a cross-sectional side elevation view of the intraosseous device according to the present disclosure during use in a first mode of operation.
FIG. 11 illustrates a cross-sectional side elevation view of the intraosseous device according to the present disclosure during use in a second mode of operation.
FIG. 12 illustrates an exploded cross-sectional side elevation view of the intraosseous device according to the present disclosure during use in a second mode of operation in which the manual intraosseous driver assembly is removed from the stabilizer assembly.
FIG. 13 illustrates a perspective view of the intraosseous device according to the present disclosure during use in a second mode of operation in which the manual intraosseous driver assembly is removed from the stabilizer assembly.
FIG. 14 illustrates a perspective view of a manual driver assembly of the intraosseous device according to the present disclosure during use in a second mode of operation.

### DETAILED DESCRIPTION

The present disclosure provides a bone-penetrating manual driver and stabilizer assembly operable to locate a suitable insertion site and penetrate the underlying bone, such as a human patient's sternum, to provide a quick and easy conduit to an intraosseous space within the bone for associated medical procedures, including delivery of fluid and medication, aspiration, and biopsy of bone marrow, among others.

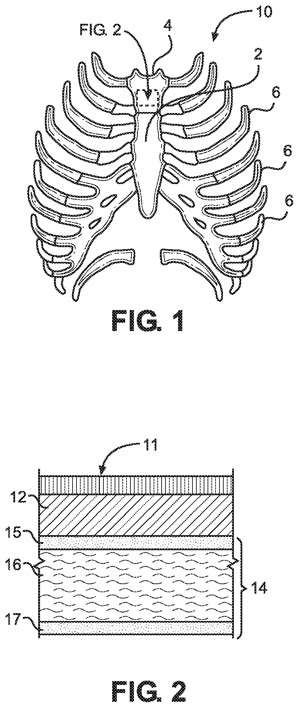

FIG. 1 depicts a schematic view of the ribcage of a human 10. The sternum 2 is a flat, narrow bone between the ribs 6 comprising three segments: the manubrium, the body, and the xiphoid process. The sternum also comprises a sternal notch 4 (also called the "suprasternal notch" or the "jugular notch"), which is a U-shaped anatomical feature located above the sternum, below the throat, and between the clavicles. FIG. 2 shows a cross-sectional view of a portion of the sternum 2. Skin 11 overlays a layer of subcutaneous tissue 12, which in turn overlays bone 14. Bone 14 includes an intraosseous space 16 bounded by anterior compact bone (i.e., anterior cortex) 15 and posterior compact bone (i.e., posterior cortex) 17. Intraosseous space 16 is the region between the anterior and posterior cortex. Bone marrow includes blood, blood forming cells, and connective tissue found in the intraosseous space.

Anterior compact bone 15 and posterior compact bone 17 are each approximately 2.0 millimeters (mm) thick and intraosseous space 16 is approximately 10.0 mm thick in most adult patients. Thus, the total thickness of bone 14 is approximately 14.0 mm. The target zone within the intraosseous space 16 is the center, which is approximately 7.0 mm from the upper surface of anterior compact bone 15 in most adult patients.

The intraosseous space 16 may be accessed by an intraosseous (IO) access device, which may include, but is not limited to, a penetrator assembly comprising a hollow needle, hollow drill bit, bone penetrator, catheter, cannula, trocar, stylet, inner penetrator, outer penetrator, needle or needle set, or other device operable to provide access to an intraosseous space or interior portions of a bone. Such IO access devices may be formed, at least in part, from metal alloys such as 304 stainless steel and other biocompatible materials associated with needles and similar medical devices. A wide variety of IO access devices may be formed in accordance with one or more teachings of the present disclosure. For instance, trocars, spindles, and/or shafts may be disposed within a cannula during insertion at a selected insertion site. Inner penetrators may include such trocars, spindles, and shafts, among others. Further, inner penetrators may comprise various lengths including, but not limited to, 20 to 50 millimeters (e.g., between 35 and 40 mm, 38.5 mm, and/or the like). Outer penetrators may include catheters, cannulas, hollow needles, and hollow drill bits, among others. In some implementations, the penetrator assembly may include a flexible outer penetrator and a rigid inner penetrator as disclosed in international patent application no. PCT/IB2019/053900.

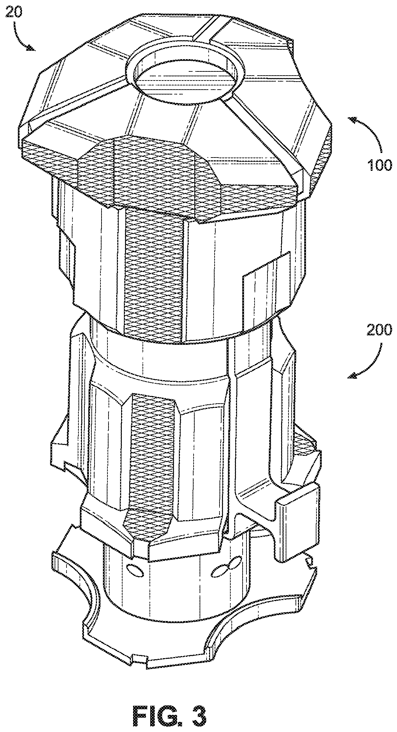

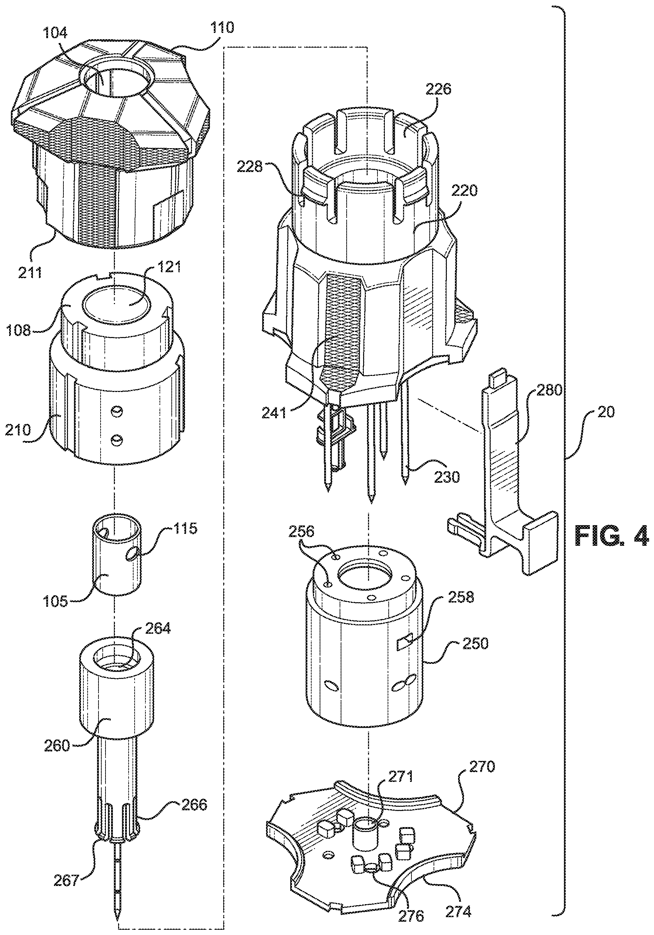

FIGS. 3 and 4 depict an implementation of an intraosseous (IO) access device 20 and its components. In a first mode of operation, the IO access device is operable to help locate a suitable insertion site and manually penetrate underlying bone, such as a patient's sternum, to quickly and easily provide a conduit to an intraosseous space within the bone for associated medical procedures, including delivery of fluid and medication, aspiration, and biopsy of bone marrow, among others. In a second mode of operation, the IO access device is operable for manual insertion into a patient's intraosseous space at a peripheral insertion site, such as a patient's humerus or tibia.

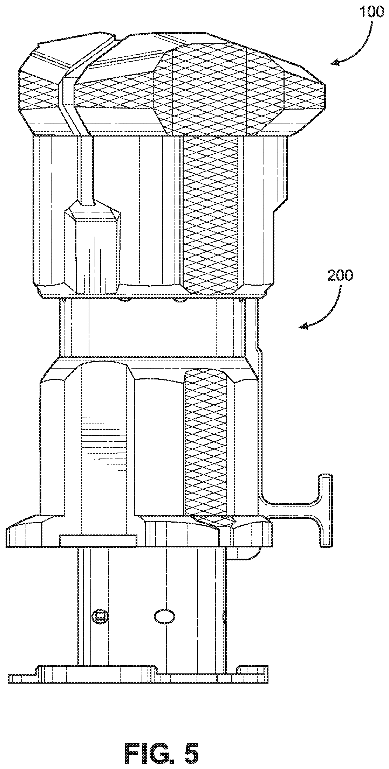

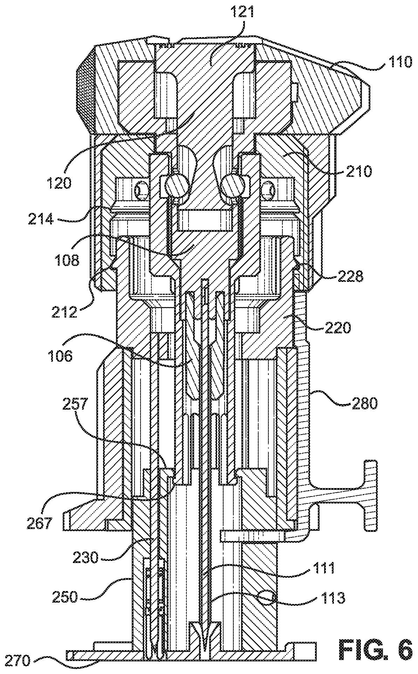

FIGS. 5 and 6, for instance, depict an intraosseous access device 20 of the present disclosure in an initial position prior to use. The intraosseous access device comprises a manual intraosseous driver assembly 100 removably coupled to a stabilizer assembly 200. The manual IO driver assembly 100 comprises a handle 110 connected to an inner penetrator hub 108, which is attached to an inner penetrator 111. The handle 110 may comprise a textured outer surface to provide an anti-slip grip for the user. The inner penetrator 111 may, for example, take the form of any suitable stylet or trocar, as previously discussed above. The inner penetrator 111 includes a distal end having a tip 102 operable to penetrate bone and associated bone marrow. The inner penetrator 111 further includes a proximal end that may have a notch 112 configured to assist in coupling the inner penetrator hub 108 to the inner penetrator 111. For instance, the inner penetrator hub 108 may be overmolded over the inner penetrator 111 such that the material from the inner penetrator hub may be molded to extend into the notch 112. The inner penetrator 111 extends from the distal end 116 of the inner penetrator hub 108.

The manual intraosseous driver assembly 100 also includes an outer penetrator hub 106 that is coupled to an outer penetrator 113. A distal end 116 of the inner penetrator hub 108 is configured to releasably engage the outer penetrator hub 106, as will be discussed in further detail below. The outer penetrator 113 may, for example, take the form of a hollow tube, such as cannula (e.g., a metal cannula), or a hollow drill bit, and which may be configured (e.g., to possess sufficient rigidity) such that the outer penetrator 113 will not buckle or otherwise be damaged as it is inserted through anterior compact bone together with the inner penetrator 111. The outer penetrator hub 106 includes a proximal end 107 and a distal end 109. The outer penetrator 113 also includes a proximal end 118 and a distal end 117, the proximal end 118 coupled to the outer penetrator hub 106. The outer penetrator distal end 117 includes a cutting surface operable to penetrate bone and associated marrow. The outer penetrator 113 extends from the distal end 109 of the outer penetrator hub 106.

The inner penetrator hub 108 is configured to removably attach to the outer penetrator hub 106. More particularly, the proximal end 107 of the outer penetrator hub 106 and the distal end 116 of the inner penetrator hub 108 may be configured as complimentary connectors (with, for example, distal end 116 being configured as a female Luer connector and proximal end 107 being configured as a male Luer connector, though these configurations could be reversed in other implementations) to allow the handle 110 to be removably coupled to the outer penetrator 113. Further, a distal end 116 of the inner penetrator hub 108 may include a male projection that is tapered to match an inwardly-tapered passageway at the proximal end 107 of the outer penetrator hub 106. In other implementations, for example, the outer penetrator hub 106 may include an internal surface or an external surface that is threaded and that is proximate a passageway that is in fluid communication with the passageway of outer penetrator. The inner penetrator hub 108 may include a complimentary external surface or internal surface that is threaded to mate with the corresponding threaded surface of the outer penetrator hub 106.

The outer penetrator 113 comprises a longitudinal passageway configured to slidably receive a portion of the inner penetrator 111 when the inner penetrator hub 108 is attached to the outer penetrator hub 106, thus forming a penetrator assembly. The handle 110 of the manual IO driver assembly is configured to manually drive the penetrator assembly into an intraosseous space, such that the handle has a shape suitable for grasping during manual insertion of the inner and outer penetrators into the bone and associated bone marrow. The handle 110 is configured to allow manual force to be applied and at the same time permit rotation of the handle during insertion of the penetrator assembly into the IO space.

When the inner penetrator hub 108 and the outer penetrator hub 106 are coupled to each other, the inner penetrator 111 is disposed within the passageway of the outer penetrator 113, and the inner penetrator tip 102 extends beyond the distal end 117 of the outer penetrator 113. The inner penetrator tip 102 and the outer penetrator distal end 117 are each operable to penetrate bone and associated bone marrow. More particularly, the inner penetrator tip 102 and the outer penetrator distal end 117 are configured to cooperate with each other to form a penetrator assembly tip operable to penetrate bone and associated bone marrow when the inner penetrator hub 108 is attached to the outer penetrator hub 106.

The tip 102 of the inner penetrator 111 is pointed and configured to allow the IO access device 20 to be driven into an intraosseous space, such as intraosseous space 16. The inner penetrator 111 fits closely within the passageway of the outer penetrator 113 such that the inner penetrator 111 prevents the outer penetrator 113 from becoming clogged with tissue (e.g., skin, bone, marrow) as the IO access device is driven into an insertion site of a subject (e.g., a patient). The inner penetrator tip 102 and the outer penetrator distal end 117 may be ground together to form corresponding cutting surfaces in some implementations where both the inner penetrator 111 and the outer penetrator 113 comprise a suitable metal. In other implementations, the inner penetrator tip 102 and the outer penetrator distal end 117 may be ground separately to form corresponding cutting surfaces configured to penetrate bone and associated marrow. Once the IO access device is properly positioned at the insertion site, the inner penetrator hub 108 can be disengaged from the outer penetrator hub 106 such that the proximal end 107 (which may take the form of a male Luer lock) is exposed and a conduit is formed from the outer penetrator hub 106 through the outer penetrator 113 to the intraosseous space. A fluid source may then be coupled to the proximal end 107 of the outer penetrator hub 106 to deliver fluid through the outer penetrator 113 into the intraosseous space.

A proximal end 119 of the inner penetrator hub 108 includes a recess 104 configured to receive an activator 120, such as a push button. The recess 104 at the proximal end 119 of the inner penetrator hub 108 is aligned with a through-hole 103 in a top surface of the handle 110 to provide access to the activator 120. Activation of the activator 120 disengages the manual IO driver assembly 100 from the stabilizer assembly 200 to switch the IO access device to the second mode of operation in which the manual driver assembly is operable for manual insertion into a patient's intraosseous space at a peripheral insertion site, as will be further described in detail below.

The stabilizer assembly 200 comprises an outer sleeve 210 configured to slidably fit over a bone probe ring 220. Both the outer sleeve 210 and the bone probe ring 220 have a generally cylindrical shape. An interior surface of the outer sleeve 210 includes a first annular detent 212 and a spaced apart second annular detent 214. The bone probe ring 220 includes a first end 222 configured to releasably engage the first annular detent 212 when the outer sleeve is in a first or undeployed position, and releasably engage the second annular detent 214 when the outer sleeve is in a second or deployed position. In particular, the first end 222 includes a plurality of circumferentially spaced apart resilient arms 226, each arm including an outwardly protruding catch 228 operable to engage the first annular detent 212 when the outer sleeve 210 is in the first position, and engage the second annular detent 214 when the outer sleeve is in the second position. The resiliency of the arms 226 allows the respective protrusions 228 to snap into engagement with the corresponding detents 212, 214. A first grip sleeve 211 may be fit over the outer sleeve to provide an anti-slip grip for the user.

The bone probe ring 220 further comprises a second end 223 including an inwardly extending flange having an underside with a plurality of openings from which respective bone probes 230 extend. For instance, the flange may include five openings corresponding to five bone probes, although other implementations may have more or less openings and corresponding bone probes. In some implementations, a single bone probe may be provided. In other implementations, a plurality of bone probes (for example 2 or 3 bone probes) may be provided. The bone probes may be arranged so that they stabilize the IO access device during insertion of a penetrator assembly into the intraosseous space at a desired location and orientation. For example, three bone probes may be arranged in a triangle surrounding the inner and outer penetrators.

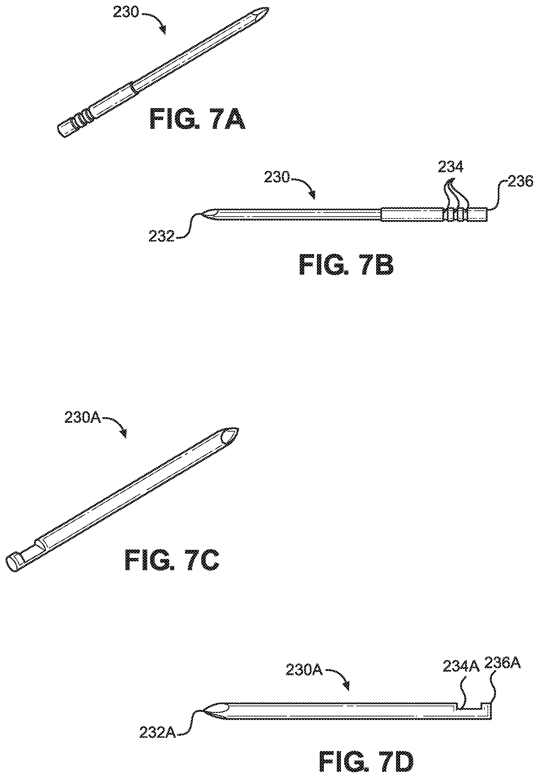

As shown in FIGS. 7A and 7B, each probe 230 comprises a pointed tip 232, a plurality of circumferential grooves or notches 234, and a proximal end 236, where the annular notches 234 are closer to the proximal end 236 than to the tip 232. The probes 230 may comprise stainless steel, though other suitable sterile or biocompatible materials (or materials capable of being made sterile before use on a patient) may be used. The proximal end 236 is configured to be inserted into the respective opening in the second end 223 of the bone probe ring 220. In some implementations, the bone probes 230 may be fixed to the bone probe ring 220, such as by being bonded using UV-curable adhesive applied to the annular grooves 234 and/or the proximal end 236 of the probe 230. In other implementations, the bone probes 230 may be force fit through the respective openings such that they are held in place by an interference fit. In still other implementations, the probes 230 may be fixed to the bone probe ring 220 as part of an injection molding process or using epoxy. FIGS. 7C and 7D depict another implementation of bone probes 230a that are suitable for use with the stabilizer assembly 200. Each probe 230a comprises a pointed tip 232a, a groove or notch 234a, and a proximal end 236a, where the notch 234a is closer to the proximal end 236a than to the tip 232a. Each probe 230a may comprise stainless steel, though other suitable sterile or biocompatible materials (or materials capable of being made sterile before use on a patient) may be used. The proximal end 236a is configured to be inserted into the respective opening in the second end 223 of the bone probe ring 220. Probes 230a may be fixed to the bone probe ring 220, such as by being bonded using UV-curable adhesive applied to the notch 234a and/or the proximal end 236a of the probe 230a. In other implementations, the probe 230a may be force fit in the respective opening in the flange of the bone probe ring 220, thus being held in place by an interference fit. In other implementations, the probes 230a may be fixed to the bone probe ring 220 as part of an injection molding process or using epoxy. Each probe 230, 230a may comprise any of various lengths.

The second end 223 of the bone probe ring 220 is connected to a stabilizer housing 240. A second grip sleeve 241 may be fit over the outer sleeve stabilizer housing 240 to provide an anti-slip grip for the user. The stabilizer housing 240 includes a first end 242 connected to the second end 223 of the bone probe ring. The stabilizer housing 240 further includes a second end 243 from which each bone probe 230 extends. The stabilizer housing 240 has an internal passageway 244 configured to slidably receive a protective shield 250.

The stabilizer assembly further comprises a retainer 260 having a first end 262 fixedly disposed within the bone probe ring 220. A second end 263 of the retainer 260 is slidably coupled to the protective shield 250. The protective shield 250 has in internal passageway 254 configured to slidably receive a portion of the retainer 260. The retainer 260 has an internal passageway 264 configured to removably receive a portion of the manual IO drier assembly 100. The first end 262 of the retainer 260 is configured to lock the manual IO driver assembly 100 to the stabilizer assembly 200. In particular, the first end 262 includes a pair of oppositely spaced apart retention seats 265. The first end 262 of the retainer 260 is configured to receive the inner penetrator hub 108. Further, a collar 105 is disposed between the inner penetrator hub 108 and the retainer 260. A pair of retention members, such as balls 124, are partly received in the respective retention seats 265 of the retention member. The balls 124 serve to couple the retainer 260 to the inner penetrator hub 108 and collar 105. The balls 124 are of a diameter slightly smaller than the retention aperture 125 of the inner penetrator hub, but are slightly larger than corresponding collar apertures 115 of the collar 105. The balls 124 therefore fit partly in the respective collar apertures 115 of the collar such that they abut the retention seat 265 of the retainer, coupling the inner penetrator 108 to the retainer 260, and thus locking the manual IO driver assembly 100 to the stabilizer assembly 200. The second end 263 of the retainer includes a plurality of resilient fingers 266 annularly disposed along a circumference of the second end 263, each finger including a respective ridge or nub 267 protruding radially outward therefrom.

The protective shield 250 includes a first end 252 having an inwardly extending annular flange 257. The protective shield 250 is operable to move from a first or extended position to provide sharps protection from the distal ends of the inner and outer penetrators 111, 113 as well as the bone probes 230, and a second or retracted position to expose the respective inner and outer penetrators 111, 113 and the bone probes 230 during an insertion procedure. The respective ridges or nubs 267 disposed on the resilient fingers 266 of the retainer 260 are operable engage the annular flange 257 of the protective shield 250 to limit how far the protective shield extends from the stabilizer housing 240 in the first or extended position. The protective shield 250 also includes a plurality of longitudinal channels 256 annularly disposed around the circumference of the shield, each channel 256 configured to slidably receive a corresponding bone probe 230. When the protective shield is in a first or extended position, the tips 232 of each bone probe 230 are disposed within the respective channels 256 to provide sharps protection. When the protective shield is in a second or retracted position, the tips 232 of each bone probe 230 extend from the respective channels 256. In some implementations, a portion of at least one of the channels 256 may include a safety clip assembly operable to secure the respective bone probe 230.

A base 270 is connected to a second end 253 of the retractable shield 250. The base comprises a guide hole 271 configured to guide the penetrator assembly during an insertion procedure. The base 270 also comprises a plurality of through-holes 276 corresponding to, and aligned with, the plurality of channels 256 of the protective shield 250. The through-holes 276 are configured to permit passage of the respective bone probes through the base 270 during an insertion procedure. The base 270 may also comprise an alignment cutout 274, such as an arc-shaped portion of the base. The alignment feature 274 is configured to approximate the shape of the sternal notch of a human patient and is operable to indicate proper placement of the base 270 against the patient. The stabilizer assembly 200 is properly located on the chest of a patient when the base 270 is placed over the sternum such that the sternal notch is visible and at least partially (and, preferably, completely) bounded by alignment cutout 274. The second end 243 of the stabilizer housing 240 and/or the second grip sleeve 241 may have a shape similar to that of base 270. This helps the user to quickly position the base 270 in appropriate alignment with a subject for insertion of a bone probe 230.

A safety latch 280 is operable to prevent the stabilizer assembly 200 from moving from the first or extended position to the second or retracted position. In particular, the safety latch 280 includes a first end 282 configured to engage the outer sleeve 210 when the outer sleeve is in the first or extended position. Further, the safety latch 280 includes a second end 283 defining a pin portion configured to engage the protective shield 250 when the protective shield is in the first or extended position. The protective shield 250 includes a side aperture 258 configured to removably receive the pin portion of the second end 283 of the latch 280. A user may remove the safety latch 280 from the stabilizer assembly 200 by pulling a tab 284 to disengage the second end 283 from the side aperture 258 of the protective shield 250, thus permitting the stabilizer assembly 200 to move to the second or retracted position.

As previously described, the IO access device 20 may be used in a first mode of operation to help locate a suitable insertion site and manually penetrate underlying bone, such as a patient's sternum, for quickly and easily providing a conduit to an intraosseous space within the bone. Prior to use, in an initial position of this first mode of operation, the manual IO driver assembly 100 is attached to the stabilizer assembly 200. Further, both the outer sleeve 210 and the protective shield 250 are in their respective first or extended positions prior to use.

Prior to use, the user first must remove the safety latch 280 from the IO access device by pulling on the tab 284, thereby disengaging the second end 283 from the side aperture 258 of the protective shield 250. In particular, the pin portion of the safety latch 280 must be removed from the side aperture 258 of the shield 250 before the IO access device can be used in an intraosseous insertion procedure. The pin prevents operation of the IO access device when inserted into the aperture 258 by blocking the shield 250 from telescoping into the stabilizer housing 240. The locking pin may be inserted during manufacture or before use of IO access device, and removed to prepare the IO access device for use.

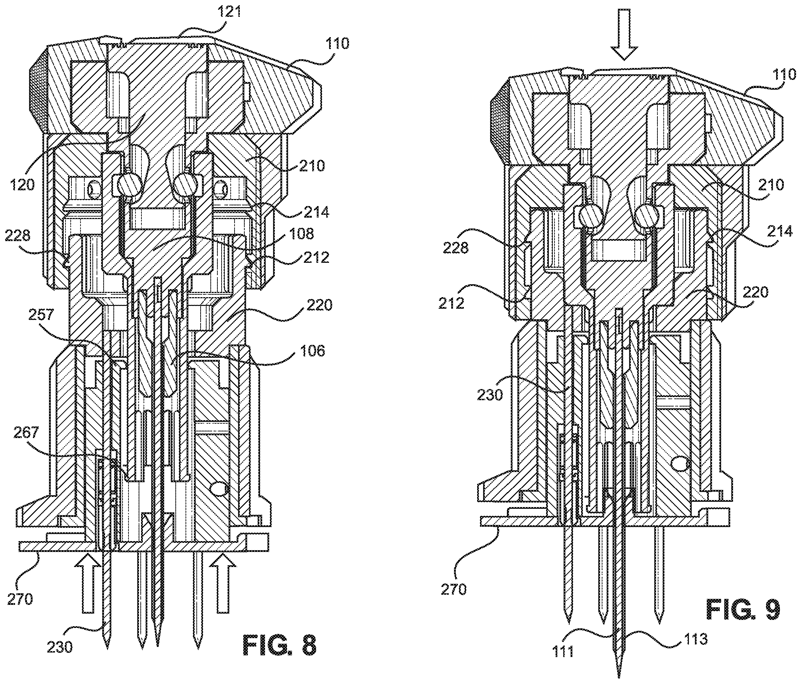

The IO access device may be operated by placing the base 270 against the skin of a patient over a bone into which it is desired to insert the penetrator assembly. The base 270 has an arc-shaped alignment cutout 274 that helps a user align the IO access device with a patient's sternal notch. In other implementations, guide features may be provided to facilitate alignment with anatomical landmarks at other infusion sites. The user operates the IO access device by first pushing on the handle 110, as depicted in FIG. 8. As the handle 110 is pushed, the shield 250 telescopes into the stabilizer housing 240 to move from its first or extended position to its second or retracted position. As the shield 250 telescopes into the stabilizer housing 240, the penetrator assembly 111, 113, as well as the surrounding bone probes 230, penetrate the patient's skin and underlying soft tissue. The base 270 assists in keeping the IO access device 20 over the desired insertion site and in the desired orientation. During use, the base 270 is substantially perpendicular to the penetrator assembly and assists in introducing the inner and outer penetrators 111, 113 straight into the patient's sternum.

A first depth of insertion of the inner and outer penetrators 111, 113 is determined when the tips 232 of the respective bone probes 230 contact the bone. At this first depth of insertion, the inner and outer penetrators 111, 113 are inserted the same distance as the bone probes 230, and therefore the inner and outer penetrators do not yet penetrate into the intraosseous space. Insertion of the inner and outer penetrators 111, 113 into the intraosseous space is then carried out by the user pushing the handle 110 again to slide the outer sleeve 210 over the bone probe ring 220 toward the stabilizer housing 240. In other words, the outer sleeve 210 is moved from its first or extended position to its second or retracted position, as depicted in FIG. 9, to deploy the inner and outer penetrators 111, 113 into the intraosseous space. As the outer sleeve 210 is moved from its first or extended position to its second or retracted position, the catch 228 on each resilient arm 226 at the first end of the bone probe ring 220 disengages the first annular detent 212 of the outer sleeve to unlock the outer sleeve from its first or extended position. The catch 228 then engages the second annular detent 214 of the bone probe ring 220 to lock the outer sleeve 210 in its second or retracted position. Each catch 228 may have a uniform but asymmetrical tooth shape having a slope on at least one edge. Similarly, the first and second annular detents 212, 214 may have a uniform but asymmetrical tooth shape having a slope on at least one edge corresponding to that of the teeth. When the outer sleeve 210 is moved from its extended position to its retracted position, the catch 228 of the bone probe ring 220 easily slide up and over the gently sloped edges of the first and second annular detents. The resilient arms 226 force the teeth of the catch into the depression between the teeth of the detents as it passes the tip portion of each tooth, thus resulting in an audible snap or click indicating to the user that the outer sleeve is locked in its second or retracted position. The outer sleeve is prevented from sliding back to its first or extended position because the catch 228 abuts against the steeply sloped edge of the second annular detent 214, thereby locking the outer sleeve in the retracted position. In some implementations, the handle 110 may be twisted to deploy the inner and outer penetrators 111, 113 into the intraosseous space.

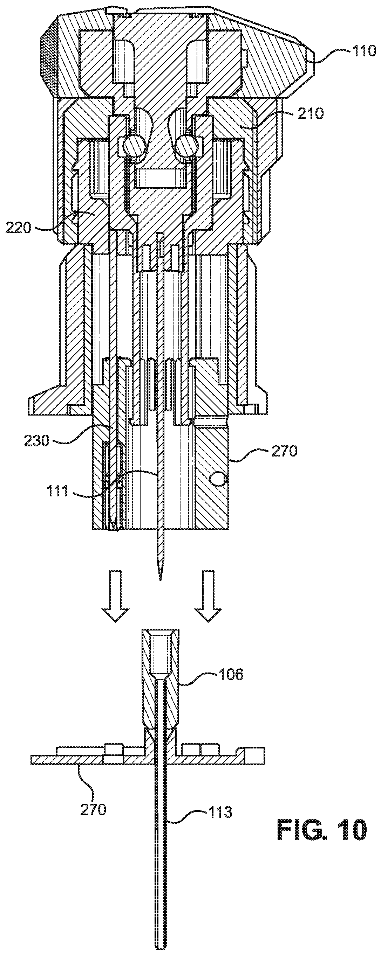

As shown in FIG. 10, once the inner and outer penetrators 111, 113 have penetrated the patient's bone to a desired depth, a release mechanism uncouples the base 270 from the protective shield. The outer penetrator hub 106 is also uncoupled form the from the inner penetrator hub 108 (and thus the outer penetrator 113 is likewise uncoupled from the inner penetrator 111). The depth is typically set so that the insertion of the inner and outer penetrators 111, 113 will stop when their tip are in the patient' bone marrow. After the release mechanism is triggered, the manual driver and stabilizer assembly may be withdrawn from the insertion site to leave in place the base 270, the outer penetrator hub 106, and the outer penetrator 113. As the stabilizer assembly 200 is removed from the insertion site, the protective shield 250 is urged outwardly from the stabilizer housing 240 back to its first or extended position so as to protect any users from inadvertent contact with the bone probes 230. The base 270 may be adhered to the patient's skin to protect the infusion site and to provide an anchor for strain relief for any tubing that may be coupled to the infusion tube assembly, or to provide strain relief for other tubing systems, catheters, or the like. Further, a flexible outer penetrator may be utilized so that it may be manipulated and fixed to the patient after the stabilizer assembly is removed in order to provide a lower profile (i.e., by bending the outer penetrator down to secure it against the skin).

In a second mode of operation, the IO access device is operable for manual insertion into a patient's intraosseous space at a peripheral insertion site. Prior to use, in an initial position of the second mode of operation, the manual IO driver assembly 100 is attached to the stabilizer assembly 200. Further, both the outer sleeve 210 and the protective shield 250 are in their respective first or extended positions prior to use. In this second mode of operation, however, the user is able to detach the manual IO driver assembly 100 from the stabilizer assembly 200 so that the manual IO driver assembly can be used without the stabilizer assembly at a peripheral insertion site.

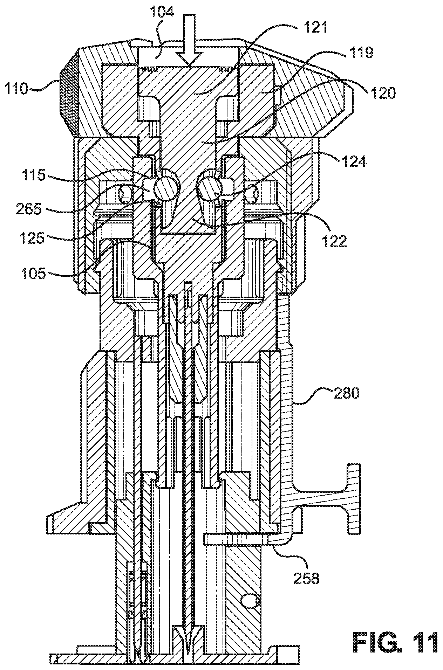

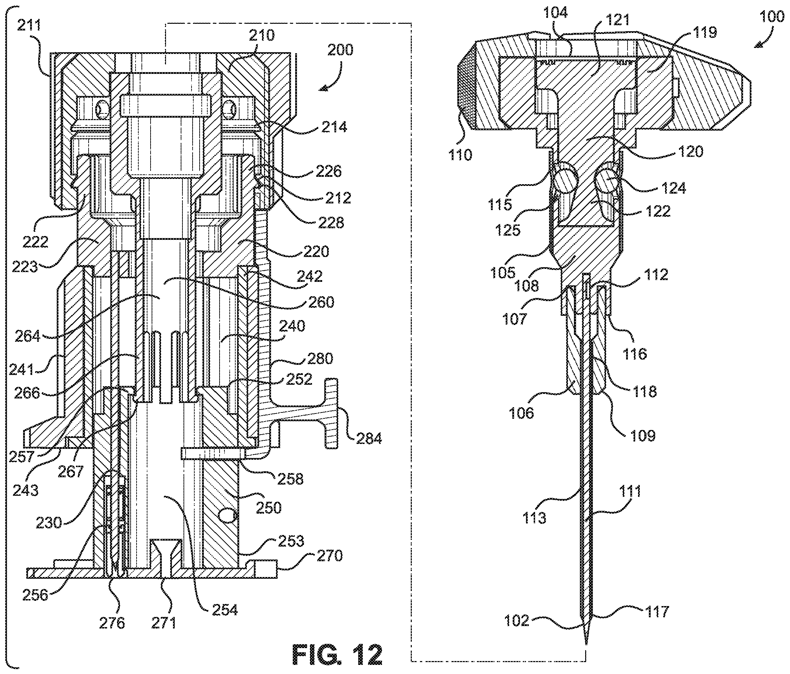

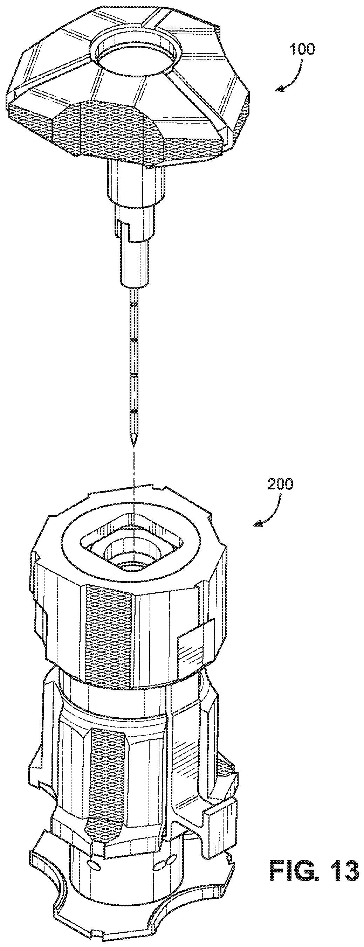

To disengage the manual IO driver assembly 100 from the stabilizer assembly 200, the user first must remove the safety latch 280 from the IO access device by pulling on the tab 284, thereby disengaging the second end 283 from the side aperture 258 of the protective shield 250. Next, the user actuates the activator 120 to begin the decoupling process. The activator 120 includes ahead portion 121 and a neck portion 122. The head portion 121 is disposed in the through-hole 103 of the handle 110, and the neck portion 122 is disposed in the recess 104 of the inner penetrator hub 108. Actuation of the activator 120 includes a user pushing down on the head portion 121, as shown in FIG. 11. A biasing member, such as a compression spring, may be provided between a bottom surface of the recess 104 and the neck portion 122 of the activator 120 to bias the activator toward a locked position. As the head portion 121 is pushed down against the biasing force of the biasing member, the neck portion 122 correspondingly moves downward further into the recess of the inner penetrator hub 108. The thin neck portion 122 aligns with the retention apertures 125 and the collar apertures 115 as it is slid downward, allowing the retention balls 124 to move into the recess 104 of the inner penetrator hub and out of the retention seat of the retainer 260, thus unlocking the inner penetrator from the retainer. Once the inner penetrator is unlocked from the retainer, the manual IO driver assembly 100 may be completely detached and removed from the stabilizer assembly 200 by pulling it out of the stabilizer, as illustrated in FIGS. 12 and 13. Consequently, the manual IO driver assembly 100 may be used for peripheral insertion of the inner and outer penetrators 111, 113 into an intraosseous space at a peripheral insertion site.

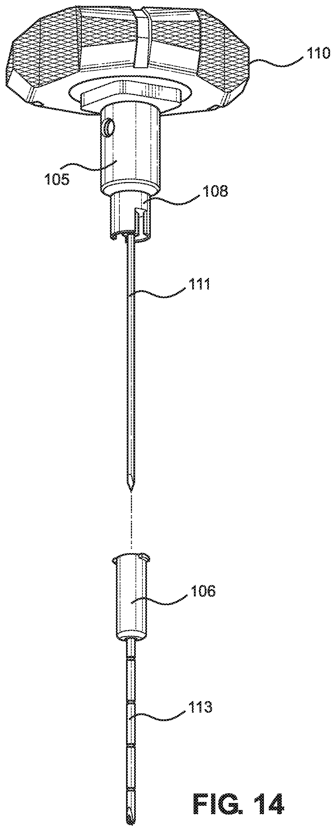

Once access to the bone marrow is achieved, the user may further detach the outer penetrator hub 106 from the inner penetrator hub 108 as shown in FIG. 14, thus leaving the outer penetrator within the intraosseous space, as previously described above. Also, as previously described above, a flexible outer penetrator may be utilized so that it may be manipulated and secured to the patient after insertion within the intraosseous space in order to provide a lower profile (i.e., by bending the outer penetrator down to secure it against the patient's skin).

While the intraosseous access device has been described in terms of what may be considered to be specific aspects, the present disclosure is not limited to the disclosed aspects. Moreover, the many features and advantages of the disclosure are apparent from the detailed specification, and thus, it is intended by the appended claims to cover all such features and advantages of the disclosure which fall within the scope of the disclosure. Further, it is not desired to limit the disclosure to the exact construction and operation illustrated and described, and accordingly, all suitable modifications and equivalents may be resorted to, falling within the scope of the disclosure. Accordingly, the present disclosure should be considered as illustrative and not restrictive. As such, this disclosure is intended to cover various modifications and similar arrangements included within the scope of the claims, which should be accorded their broadest interpretation so as to encompass all such modifications and similar structures.

While the intraosseous access device has been described in terms of what may be considered to be specific aspects, the present disclosure is not limited to the disclosed aspects. Moreover, the many features and advantages of the disclosure are apparent from the detailed specification.

Further, it is not desired to limit the disclosure to the exact construction and operation illustrated and described, and accordingly, all suitable modifications and equivalents may be resorted to, falling within the scope of the disclosure. Accordingly, the present disclosure should be considered as illustrative and not restrictive. The scope of the invention is defined by the claims.

## Claims

1. An intraosseous access device comprising:
a manual intraosseous driver (100) including a handle (110), an activator (120), and a penetrator assembly (111,113), the penetrator assembly having a sharp penetrating end configured to penetrate a bone and associated bone marrow; and
a stabilizer (200) including a retainer (260) and a stabilizer housing (240);
the retainer (260) having a first retainer end (262), a second retainer end (263), and an internal passageway (264) extending from the first retainer end (262) to the second retainer end (263), the internal passageway (264) configured to removably receive a portion of the manual intraosseous driver (100); and
the stabilizer housing (240) having a first housing end (242), a second housing end (243), and an internal housing section configured to receive a portion of the retainer (260);
where the activator (120) is configured to move to a locked position to lock the manual intraosseous driver (100) to the stabilizer (200) for intraosseous insertion of the penetrator assembly in a first mode of operation, and the penetrator assembly is operable to provide access to a sternal intraosseous space when the manual driver (100) is coupled to the stabilizer (200) in the first mode of operation; and
where the activator (120) is configured to move to an unlocked position to unlock the manual intraosseous driver (100) from the stabilizer (200) for intraosseous insertion of the penetrator assembly in a second mode of operation, and the penetrator assembly is operable to provide access to a peripheral intraosseous space when the manual driver (100) is decoupled from the stabilizer (200) in the second mode of operation.

2. The intraosseous access device according to claim 1, wherein the stabilizer (200) further comprises a protective shield (250) slidably disposed in the internal housing section of the stabilizer housing (240), the protective shield (250) configured to move between an extended position and a retracted position during the first mode of operation.

3. The intraosseous access device according to claim 2, wherein the extended position of the protective shield (250) is operable to provide sharps protection from the sharp penetrating end of the penetrating assembly, and wherein the retracted position of the protective shield (250) is operable to expose the sharp penetrating end of the penetrating assembly to permit insertion of the penetrating assembly into the bone and associated bone marrow.

4. The intraosseous access device according to any one of claims 2 and 3, wherein the protective shield (250) includes a first shield end slidably coupled to the second retainer end of the retainer (260).

5. The intraosseous access device according to any one of claims 2-4, wherein the stabilizer (200) further comprises a bone probe ring (220) having a first ring end, a second ring end coupled to the first housing end of the stabilizer housing (240), and a bone probe (230) extending from the second ring end, the bone probe (230) including a bone probe tip (232) operable to penetrate skin and subcutaneous tissue.

6. The intraosseous access device according to any one of claims 2-5, wherein the protective shield (250) includes a longitudinal channel configured to slidably receive the bone probe (230).

7. The intraosseous access device according to any one of claims 2-6, wherein the bone probe tip (232) of the bone probe (230) is disposed within the longitudinal channel (256) to provide sharps protection when the protective shield (250) is in the extended position, and wherein the bone probe tip (232) of the bone probe (230) extends from the longitudinal channel (256) when the protective shield (250) is in the retracted position.

8. The intraosseous access device according to any one of claims 5-7, wherein the stabilizer (200) further comprises an outer sleeve (210) slidably coupled to the bone probe ring (220), the outer sleeve (210) operable to move from an undeployed position to a deployed position in the first mode of operation.

9. The intraosseous access device according to claim 8, wherein the undeployed position of the outer sleeve (210) permits the penetrator assembly to penetrate the skin and subcutaneous tissue, and where the deployed position of the outer sleeve (210) permits the penetrator assembly to penetrate the bone and associated bone marrow.

10. The intraosseous access device according to claim 9, wherein the outer sleeve (210) includes a first detent (212) and a second detent (214), the first detent spaced apart from the second detent; and wherein the bone probe ring (220) is configured to releasably engage the first detent when the outer sleeve (210) is in the undeployed position, and wherein the bone probe ring (220) is configured to releasably engage the second detent when the outer sleeve (210) is in the deployed position.

11. The intraosseous access device according to claim 10, wherein the first ring end of the bone probe ring (220) comprises a resilient arm including and outwardly protruding catch operable to engage the first detent (212) when the outer sleeve (210) is in the undeployed position and operable to engage the second detent (214) when the outer sleeve (210) is in the deployed position.

12. The intraosseous access device according to any one of claims 2-11, wherein the stabilizer (200) further comprises a stabilizing base connected to the protective shield (250).

13. The intraosseous access device according to any one of claims 8-12, further comprising a safety latch (280) operable to prevent the outer sleeve (210) from moving from the undeployed position to the deployed position during the first mode of operation, and operable to prevent the protective shield (250) from moving from the extended position to the retracted position during the first mode of operation.

14. The intraosseous access device according to any one of the preceding claims, wherein the first retainer end of the retainer (260) is configured to releasably secure the manual intraosseous driver (100) to the stabilizer (200).

## Patentansprüche

1. Vorrichtung für den intraossären Zugang, die Folgendes umfasst:
einen manuellen intraossären Driver (100) mit einem Griff (110), einem Aktivator (120) und einer Eindringkörperbaugruppe (111, 113), wobei die Eindringkörperbaugruppe ein scharfes Eindringende aufweist, das so ausgelegt ist, dass es in einen Knochen und zugehöriges Knochenmark eindringen kann, und
einen Stabilisator (200) mit einer Halterung (260) und einem Stabilisatorgehäuse (240),
wobei die Halterung (260) ein erstes Halterungsende (262), ein zweites Halterungsende (263) und einen Innendurchgang (264) aufweist, der sich von dem ersten Halterungsende (262) bis zu dem zweiten Halterungsende (263) erstreckt, wobei der Innendurchgang (264) so ausgelegt ist, dass er einen Teil des manuellen intraossären Drivers (100) abnehmbar aufnehmen kann, und
wobei das Stabilisatorgehäuse (240) ein erstes Gehäuseende (242), ein zweites Gehäuseende (243) und einen Innengehäuseabschnitt aufweist, der so ausgelegt ist, dass er einen Teil der Halterung (260) aufnehmen kann,
wobei der Aktivator (120) so ausgelegt ist, dass er sich in eine Verriegelungsposition bewegt und so den manuellen intraossären Driver (100) für das intraossäre Einführen der Eindringkörperbaugruppe in einem ersten Betriebsmodus mit dem Stabilisator (200) verriegelt, und die Eindringkörperbaugruppe dazu dient, Zugang zu einem sternalen Intraossärraum zu verschaffen, wenn der manuelle Driver (100) im ersten Betriebsmodus mit dem Stabilisator (200) gekoppelt ist, und
wobei der Aktivator (120) so ausgelegt ist, dass er sich in eine Entriegelungsposition bewegt und so den manuellen intraossären Driver (100) für das intraossäre Einführen der Eindringkörperbaugruppe in einem zweiten Betriebsmodus von dem Stabilisator (200) entriegelt, und die Eindringkörperbaugruppe dazu dient, Zugang zu einem peripheren Intraossärraum zu verschaffen, wenn der manuelle Driver (100) im zweiten Betriebsmodus von dem Stabilisator (200) entkoppelt ist.

2. Vorrichtung für den intraossären Zugang nach Anspruch 1, wobei der Stabilisator (200) ferner einen Schutzschild (250) umfasst, der verschiebbar in dem Innengehäuseabschnitt des Stabilisatorgehäuses (240) angeordnet ist, wobei der Schutzschild (250) so ausgelegt ist, dass er sich im ersten Betriebsmodus zwischen einer ausgefahrenen und einer eingefahrenen Position bewegen kann.

3. Vorrichtung für den intraossären Zugang nach Anspruch 2, wobei die ausgefahrene Position des Schutzschilds (250) zum Bieten von Schutz vor Verletzungen durch das scharfe Eindringende der Eindringkörperbaugruppe und die eingefahrene Position des Schutzschildes (250) zum Freilegen des scharfen Eindringendes der Eindringkörperbaugruppe dient, damit diese in den Knochen und das zugehörige Knochenmark eingeführt werden kann.

4. Vorrichtung für den intraossären Zugang nach einem der Ansprüche 2 und 3, wobei der Schutzschild (250) ein erstes Schildende aufweist, das verschiebbar mit dem zweiten Halterungsende der Halterung (260) gekoppelt ist.

5. Vorrichtung für den intraossären Zugang nach einem der Ansprüche 2-4, wobei der Stabilisator (200) ferner einen Knochensondenring (220) mit einem ersten Ringende, einem mit dem ersten Gehäuseende des Stabilisatorgehäuses (240) gekoppelten zweiten Ringende und einer sich von dem zweiten Ringende aus erstreckenden Knochensonde (230) umfasst, wobei die Knochensonde (230) eine Knochensondenspitze (232) aufweist, die zum Eindringen in Haut und subkutanes Gewebe dient.

6. Vorrichtung für den intraossären Zugang nach einem der Ansprüche 2-5, wobei der Schutzschild (250) einen längs verlaufenden Kanal aufweist, der so ausgelegt ist, dass er die Knochensonde (230) verschiebbar aufnehmen kann.

7. Vorrichtung für den intraossären Zugang nach einem der Ansprüche 2-6, wobei die Knochensondenspitze (232) der Knochensonde (230) in dem längs verlaufenden Kanal (256) angeordnet ist und so Schutz vor Verletzungen bietet, wenn sich der Schutzschild (250) in der ausgefahrenen Position befindet, und wobei sich die Knochensondenspitze (232) der Knochensonde (230) aus dem längs verlaufenden Kanal (256) erstreckt, wenn sich der Schutzschild (250) in der eingefahrenen Position befindet.

8. Vorrichtung für den intraossären Zugang nach einem der Ansprüche 5-7, wobei der Stabilisator (200) ferner eine Außenhülse (210) umfasst, die verschiebbar mit dem Knochensondenring (220) gekoppelt ist, wobei die Außenhülse (210) im ersten Betriebsmodus aus einer Ruheposition in eine Einsatzposition bewegt werden kann.

9. Vorrichtung für den intraossären Zugang nach Anspruch 8, wobei die Ruheposition der Außenhülse (210) der Eindringkörperbaugruppe ermöglicht, in die Haut und subkutanes Gewebe einzudringen, und die Einsatzposition der Außenhülse (210) der Eindringkörperbaugruppe ermöglicht, in den Knochen und das zugehörige Knochenmark einzudringen.

10. Vorrichtung für den intraossären Zugang nach Anspruch 9, wobei die Außenhülse (210) eine erste Sperre (212) und eine zweite Sperre (214) aufweist, wobei die erste Sperre von der zweiten beabstandet ist, und wobei der Knochensondenring (220) so ausgelegt ist, dass er lösbar in die erste Sperre einrastet, wenn sich die Außenhülse (210) in der Ruheposition befindet, und wobei der Knochensondenring (220) so ausgelegt ist, dass er lösbar in die zweite Sperre einrastet, wenn sich die Außenhülse (210) in der Einsatzposition befindet.

11. Vorrichtung für den intraossären Zugang nach Anspruch 10, wobei das erste Ringende des Knochensondenrings (220) einen elastischen Arm mit einer nach außen vorstehenden Nase umfasst, die in die erste Sperre (212) einrastet, wenn sich die Außenhülse (210) in der Ruheposition befindet, und in die zweite Sperre (214) einrastet, wenn sich die Außenhülse (210) in der Einsatzposition befindet.

12. Vorrichtung für den intraossären Zugang nach einem der Ansprüche 2-11, wobei der Stabilisator (200) ferner einen stabilisierenden Fuß umfasst, der mit dem Schutzschild (250) verbunden ist.

13. Vorrichtung für den intraossären Zugang nach einem der Ansprüche 8-12, die ferner eine Sicherheitsverriegelung (280) umfasst, die verhindert, dass sich im ersten Betriebsmodus die Außenhülse (210) aus der Ruheposition in die Einsatzposition und der Schutzschild (250) aus der ausgefahrenen Position in die eingefahrene Position bewegt.

14. Vorrichtung für den intraossären Zugang nach einem der vorhergehenden Ansprüche, wobei das erste Halterungsende der Halterung (260) so ausgelegt ist, dass es den manuellen intraossären Driver (100) lösbar an dem Stabilisator (200) befestigt.

## Revendications

1. Dispositif d'accès intra-osseux comprenant :
un dispositif d'entraînement intra-osseux manuel (100) incluant une poignée (110), un activateur (120), et un ensemble de pénétrateur (111, 113), l'ensemble de pénétrateur ayant une extrémité de pénétration tranchante configurée pour pénétrer dans un os et la moelle osseuse associée ; et
un stabilisateur (200) incluant un élément de retenue (260) et un logement de stabilisateur (240) ;
l'élément de retenue (260) ayant une première extrémité d'élément de retenue (262), une deuxième extrémité d'élément de retenue (263), et une voie de passage interne (264) s'étendant de la première extrémité d'élément de retenue (262) à la deuxième extrémité d'élément de retenue (263), la voie de passage interne (264) configurée pour recevoir de manière amovible une partie du dispositif d'entraînement intra-osseux manuel (100) ; et
le logement de stabilisateur (240) ayant une première extrémité de logement (242), une deuxième extrémité de logement (243), et une section de logement interne configurée pour recevoir une partie de l'élément de retenue (260) ;
où l'activateur (120) est configuré pour se déplacer vers une position verrouillée pour verrouiller le dispositif d'entraînement intra-osseux manuel (100) avec le stabilisateur (200) pour l'insertion intra-osseuse de l'ensemble de pénétrateur dans un premier mode de fonctionnement, et l'ensemble de pénétrateur est capable de fonctionner pour fournir un accès à un espace intra-osseux sternal quand le dispositif d'entraînement manuel (100) est couplé au stabilisateur (200) dans le premier mode de fonctionnement ; et
où l'activateur (120) est configuré pour se déplacer vers une position déverrouillée pour déverrouiller le dispositif d'entraînement intra-osseux manuel (100) d'avec le stabilisateur (200) pour l'insertion intra-osseuse de l'ensemble de pénétrateur dans un deuxième mode de fonctionnement, et l'ensemble de pénétrateur est capable de fonctionner pour fournir un accès à un espace intra-osseux périphérique quand le dispositif d'entraînement manuel (100) est dissocié du stabilisateur (200) dans le deuxième mode de fonctionnement.

2. Dispositif d'accès intra-osseux selon la revendication 1, dans lequel le stabilisateur (200) comprend en outre un écran protecteur (250) disposé de manière coulissante dans la section de logement interne du logement de stabilisateur (240), l'écran protecteur (250) configuré pour se déplacer entre une position étendue et une position rétractée pendant le premier mode de fonctionnement.

3. Dispositif d'accès intra-osseux selon la revendication 2, dans lequel la position étendue de l'écran protecteur (250) est capable de fonctionner pour fournir une protection contre les objets tranchants contre l'extrémité de pénétration tranchante de l'ensemble de pénétration, et dans lequel la position rétractée de l'écran protecteur (250) est capable de fonctionner pour exposer l'extrémité de pénétration tranchante de l'ensemble de pénétration pour permettre l'insertion de l'ensemble de pénétration jusque dans l'os et la moelle osseuse associée.

4. Dispositif d'accès intra-osseux selon l'une quelconque des revendications 2 et 3, dans lequel l'écran protecteur (250) inclut une première extrémité d'écran couplée de manière coulissante à la deuxième extrémité d'élément de retenue de l'élément de retenue (260).

5. Dispositif d'accès intra-osseux selon l'une quelconque des revendications 2 à 4, dans lequel le stabilisateur (200) comprend en outre une bague de sonde osseuse (220) ayant une première extrémité de bague, une deuxième extrémité de bague couplée à la première extrémité de logement du logement de stabilisateur (240), et une sonde osseuse (230) s'étendant depuis la deuxième extrémité de bague, la sonde osseuse (230) incluant une pointe de sonde osseuse (232) capable de fonctionner pour pénétrer dans la peau et le tissu sous-cutané.

6. Dispositif d'accès intra-osseux selon l'une quelconque des revendications 2 à 5, dans lequel l'écran protecteur (250) inclut un canal longitudinal configuré pour recevoir de manière coulissante la sonde osseuse (230).

7. Dispositif d'accès intra-osseux selon l'une quelconque des revendications 2 à 6, dans lequel la pointe de sonde osseuse (232) de la sonde osseuse (230) est disposée au sein du canal longitudinal (256) pour fournir une protection contre les objets tranchants quand l'écran protecteur (250) est dans la position étendue, et dans lequel la pointe de sonde osseuse (232) de la sonde osseuse (230) s'étend depuis le canal longitudinal (256) quand l'écran protecteur (250) est dans la position rétractée.

8. Dispositif d'accès intra-osseux selon l'une quelconque des revendications 5 à 7, dans lequel le stabilisateur (200) comprend en outre un manchon externe (210) couplé de manière coulissante à la bague de sonde osseuse (220), le manchon externe (210) capable de fonctionner pour se déplacer d'une position non déployée à une position déployée dans le premier mode de fonctionnement.

9. Dispositif d'accès intra-osseux selon la revendication 8, dans lequel la position non déployée du manchon externe (210) permet que l'ensemble de pénétrateur pénètre dans la peau et le tissu sous-cutané, et où la position déployée du manchon externe (210) permet que l'ensemble de pénétrateur pénètre dans l'os et la moelle osseuse associée.

10. Dispositif d'accès intra-osseux selon la revendication 9, dans lequel le manchon externe (210) inclut une première encoche (212) et une deuxième encoche (214), la première encoche espacée de la deuxième encoche ; et dans lequel la bague de sonde osseuse (220) est configurée pour entrer en prise de manière libérable avec la première encoche quand le manchon externe (210) est dans la position non déployée, et dans lequel la bague de sonde osseuse (220) est configurée pour entrer en prise de manière libérable avec la deuxième encoche quand le manchon externe (210) est dans la position déployée.

11. Dispositif d'accès intra-osseux selon la revendication 10, dans lequel la première extrémité de bague de la bague de sonde osseuse (220) comprend un bras résilient incluant un cliquet faisant saillie vers l'extérieur capable de fonctionner pour entrer en prise avec la première encoche (212) quand le manchon externe (210) est dans la position non déployée et capable de fonctionner pour entrer en prise avec la deuxième encoche (214) quand le manchon externe (210) est dans la position déployée.

12. Dispositif d'accès intra-osseux selon l'une quelconque des revendications 2 à 11, dans lequel le stabilisateur (200) comprend en outre une base de stabilisation connectée à l'écran protecteur (250).

13. Dispositif d'accès intra-osseux selon l'une quelconque des revendications 8 à 12, comprenant en outre un loquet de sécurité (280) capable de fonctionner pour empêcher que le manchon externe (210) ne se déplace de la position non déployée à la position déployée pendant le premier mode de fonctionnement, et capable de fonctionner pour empêcher que l'écran protecteur (250) ne se déplace de la position étendue à la position rétractée pendant le premier mode de fonctionnement.

14. Dispositif d'accès intra-osseux selon l'une quelconque des revendications précédentes, dans lequel la première extrémité d'élément de retenue de l'élément de retenue (260) est configurée pour fixer de manière libérable le dispositif d'entraînement intra-osseux manuel (100) au stabilisateur (200).
